# EUROPEAN PATENT APPLICATION

(11) **EP 4 079 843 A1**
(43) Date of publication of application: **26.10.2022**
(21) Application number: 20900822.6
(22) Date of filing: 17.12.2020
(51) Int. Cl.: C12N 5/079, C12N 5/071

(54) **DIFFERENTIATION METHOD FOR PROCURING LARGE AMOUNT OF OLIGODENDROCYTES BY DISASSEMBLING 3D ORGANOIDS GENERATED FROM HUMAN PLURIPOTENT STEM CELLS**

(30) Priority: 17.12.2019 KR 20190168517
(71) Applicant: Corestem Co., Ltd., Seongnam-si, Gyeonggi-do 13486 (KR)
(72) Inventor: LEE, Sang Hun, Seoul 04763 (KR); CHANG, Mi Yoon, Seoul 04763 (KR); WOO, Hye Ji, Seoul 04763 (KR)
(74) Representative: HGF
(86) International application number: PCT/KR2020/018575
(87) International publication number: WO 2021/125844

(57) **Abstract**

The present invention relates to a method, which patterns 3D organoids prepared from human pluripotent stem cells and chops the same so as to culture oligodendrocyte progenitor cells, and induces the differentiation thereof so as to obtain a large quantity of finally differentiated oligodendrocytes. Compared to cells differentiated by a conventional differentiation method, oligodendrocytes obtained in a large quantity have the same or superior reproducibility, stability, and functionality and have remarkably shortened differentiation time, and thus are expected to be very useful for cell therapeutic agents or for screening for therapeutic drugs.

## Description

### Technical Field

The present invention relates to a method for differentiation of oligodendrocytes in a large quantity by separating 3D organoids prepared from human pluripotent stem cells and chopping the same so as to obtain a large quantity of oligodendrocyte progenitor cells.

### Background Art

Nervous system inflammatory diseases having oligodendrocytopathy include multiple sclerosis, multiple system atrophy (MSA), and the like. Representatively, multiple system atrophy is a disease that does not have a clear therapeutic agent and also shows a rapid clinical course. In addition, multiple system atrophy is a disease suitable for stem cell transplantation treatment because the lesion site is different and clear depending on the type. For the cell therapy of such oligodendrocytopathy disease, a technology to differentiate from stem cells into human oligodendrocytes is required, and protocols have been developed to differentiate from human embryonic stem cells (hESCs) or human pluripotent stem cells (hiPSCs) into oligodendrocytes. However, the differentiation methods developed up to now have difficulties in clinical application and use due to the low yield and the absence of an efficient differentiation method.

In addition, *in vivo* studies are being conducted using rats, mice, or the like in order to study oligodendrocytopathy diseases, but such an animal model is different from the actual human brain environment and there is a difference in genetic expression, so it cannot be considered suitable for research targeting human diseases.

Methods for differentiation from human pluripotent stem cells into oligodendrocytes have been developed, but the following problems have been presented.
1) The yield of oligodendrocytes that can be obtained by differentiation from human pluripotent stem cells into oligodendrocytes is low. That is, since the methods developed up to now induce differentiation into oligodendrocytes directly without a step capable of proliferation in the middle, there is a limit to the amount of oligodendrocytes that can be obtained through one differentiation.
2) It takes a long time to differentiate from human pluripotent stem cells into oligodendrocytes. In the differentiation methods developed up to now, it takes from at least 95 days to more than 200 days for the final differentiation from stem cells into oligodendrocytes. In addition, since it is a protocol that directly differentiates into oligodendrocytes without a step capable of proliferation, there is no step in which oligodendrocyte progenitor cells can be frozen as a stock and used immediately when needed.
3) There is actually no multiple system atrophy disease model using human cells. Up to now, there is no *ex vivo* multiple system atrophy (MSA) pathology model using oligodendrocytes differentiated from human pluripotent stem cells. Therefore, it is difficult to study the pathology of multiple system atrophy in the human cellular environment rather than the animal cellular environment.

### Prior Art Document

### Non-Patent Document

(Non-Patent Document 1) Differentiation of human oligodendrocytes from pluripotent stem cells, Nature Protocols. 2009;4(11): 1614-22.
(Non-Patent Document 2) Human iPSC-Derived Oligodendrocyte Progenitor Cells Can Myelinate and Rescue a Mouse Model of Congenital Hypomyelination, Cell Stem Cell Volume 12, Issue 2 Pages 139-264 (7 February 2013)
(Non-Patent Document 3) Efficient Generation of Myelinating Oligodendrocytes from Primary Progressive Multiple Sclerosis Patients by Induced Pluripotent Stem Cells, Stem Cell Reports, VOLUME 3, ISSUE 2, P250-259, AUGUST 12, 2014
(Non-Patent Document 4) Differentiation and maturation of oligodendrocytes in human three-dimensional neural cultures, Nature neuroscience, 2019 Mar;22(3):484-491.

### Detailed Description of Invention

### Technical Problem

Accordingly, as a result of research efforts in order to solve the above problems, the present inventors have developed, under the assumption that the cells extracted from the three-dimensional culture will be functionally superior to the existing two-dimensional cultured cells, a new method for differentiation of human pluripotent stem cell derived oligodendrocytes (hPSC-oligodendrocytes), which prepares a ventral patterning organoid, in which a specific patterning is generated in a manner that may contain a large number of oligodendrocyte progenitor cells (OPCs), and chops the prepared organoid, proliferates the culture, and induces differentiation into oligodendrocytes therefrom. Based on the above, the present inventors completed the present invention.

### Solution to Problem

Therefore, an object of the present invention is to provide a differentiation method, which patterns 3D organoids prepared from human pluripotent stem cells and chops the same so as to culture oligodendrocyte progenitor cells, and induces the differentiation thereof so as to obtain a large quantity of finally differentiated oligodendrocytes.

In addition, another object of the present invention is to provide a cell therapeutic agent comprising the differentiated oligodendrocytes obtained by the method as an active ingredient.

In addition, another object of the present invention is to provide a drug screening method using the differentiated oligodendrocytes obtained by the method.

### Effects of Invention

In the present invention, the finally differentiated oligodendrocytes may be differentiated in a large quantity by chopping the 3D organoids prepared from human pluripotent stem cells presented above, and separating oligodendrocyte progenitor cells from the organoids and culturing them to proliferate them, and thus a large quantity of cells may be obtained at once. For example, the differentiation method of the present invention may be easily reproduced because it may easily induce differentiation according to cell lines or without batch-to-batch variation. In addition, the differentiation method of the present invention may produce oligodendrocytes faster than the methods developed up to now, and may freeze and store oligodendrocyte progenitor cells as a stock in a step capable of proliferation and culture in the middle. In addition, when necessary, oligodendrocyte progenitor cells may be obtained immediately and may be used immediately after differentiation into oligodendrocytes in about 2-3 weeks, and thus the total time of differentiation into hPSC-oligodendrocytes may be reduced to 6-8 weeks. In other existing reports, the total differentiation time is about 10 to 20 weeks, so relatively rapid differentiation is possible (Non-Patent Documents 1 to 4). In particular, since it is possible to construct an *ex vivo* multiple system atrophy (MSA) pathology model using oligodendrocytes differentiated from human pluripotent stem cells for the first time, it is possible to study the pathology of multiple system atrophy in an actual human cellular environment.

### Brief Description of Drawings

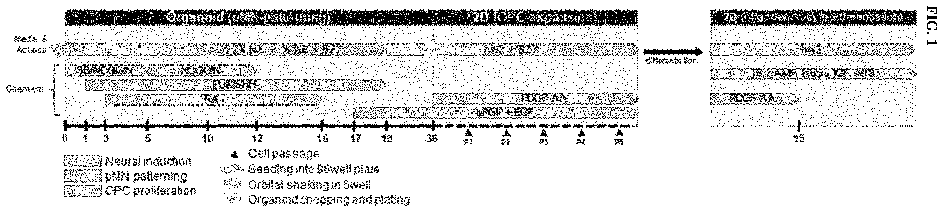

FIG. 1 schematically illustrates a protocol for differentiating oligodendrocytes using the 3D organoids according to one embodiment of the present invention.
FIG. 2 is a photograph confirming the preparation of ventral patterning organoids in two batches under an optical microscope.
FIG. 3 is a graph showing the expression of NKX2.2, Olig2, NG2, O4, PDGFRa, SOX10, and the like as mRNA markers of oligodendrocyte progenitor cells through qPCR of the patterned organoids.
FIG. 4 illustrates a result obtained by confirming the expression of marker OLIG2 of oligodendrocyte progenitor cells in cryosections of organoids by immunocytochemistry method.
FIG. 5 is a graph showing the number of cells proliferated after converting the organoids into a 2D culture environment through a physical chopping process (chopping).
FIG. 6 illustrates a result obtained by confirming that when the differentiation of oligodendrocyte progenitor cells was induced after thawing (right) compared to before cryopreservation (left), their differentiation ability was maintained and they were differentiated into oligodendrocytes expressing the marker MBP.
FIG. 7 illustrates a result obtained by confirming the marker OLIG2 that oligodendrocyte progenitor cells can be proliferated, which maintain their ability to differentiate into oligodendrocytes until 5 passages.
FIG. 8 illustrates a result obtained by confirming the oligodendrocyte progenitor cell maintenance markers OLIG2, A2B5, and PDGFRa.
FIG. 9 illustrates a result obtained by confirming the neuron bundle marker NF and the mature oligodendrocyte marker MBP that after differentiation of oligodendrocyte progenitor cells, they normally differentiate into oligodendrocytes, thereby performing the function of myelination of neurons.
FIG. 10 illustrates a result obtained by confirming the mature oligodendrocyte marker MBP.
FIG. 11 illustrates a result obtained by confirming the GFP marker that a lentivirus (pEF1α-α-syn-GFP) overexpressing alpha-synclein (synclein), which can cause synucleinopathy, is well introduced into oligodendrocyte progenitor cells.
FIG. 12 illustrates a result obtained by confirming that after transducing oligodendrocyte progenitor cells with a lentivirus (pEF1α-α-syn-GFP) overexpressing alpha-synclein, the alpha-synclein expressed in the cells is a pathological protein that is not cleaved by PK (proteinase K) compared to the monomeric form of alpha-synclein (O8, O11, and 012 compared to 100 µg M).
FIG. 13 illustrates a pattern of uptake of alpha-synclein PFF (pre-formed fibrils) by day for each cell type in oligodendrocytes compared to each neuron and astrocyte.
FIG. 14 illustrates a result obtained by confirming the degree of PFF uptake of oligodendrocytes and a-syn aggregation in oligodendrocytes by western blot.

### Best Mode for Carrying out the Invention

Hereinafter, the present invention will be described in more detail.

The present invention relates to a differentiation method, which patterns 3D organoids prepared from human pluripotent stem cells and chops the same so as to culture oligodendrocyte progenitor cells, and induces the differentiation thereof so as to obtain a large quantity of finally differentiated oligodendrocytes.

As used herein, the term "pluripotent stem cell (PSC)" refers to a stem cell capable of inducing differentiation into any type of cell constituting the body, and the pluripotent stem cell includes an embryonic stem cell (ESC) and an induced pluripotent stem cell (Ipsc, dedifferentiated stem cell).

As used herein, the term "organoid" refers to a 'mini-organ like' made to have a minimum function using stem cells, and is characterized in that it is made in a three-dimensional structure and may create an environment similar to an actual body organ even in a laboratory. That is, "organoid" refers to a cell having a 3D stereostructure, and refers to a model similar to organs such as nerves and intestines prepared through an artificial culture process that is not collected or acquired from animals and the like. The origin of the cells constituting it is not limited. The organoid may have an environment that is allowed to interact with the surrounding environment in the process of cell growth. Unlike 2D culture, 3D cell culture allows cells to grow in all directions *ex vivo.* Accordingly, in the present invention, the 3D organoid may be an excellent model for observing the development of therapeutic agents for diseases and the like by almost completely mimicking the organs that actually interact *in vivo.*

An organoid may generally be prepared by culturing human pluripotent stem cells. Specifically, it may differentiate from induced pluripotent stem cells derived from Parkinson's disease into neuroectodermal spheres.

As used herein, the term "differentiation" refers to a phenomenon in which the structure or function of cells is specialized during division and proliferation of cells and growth of the entire individual. In other words, it refers to a process of a change into a suitable form and function of cells, tissues, and the like of organisms in order to perform their respective given roles. For example, differentiation may include a process in which pluripotent stem cells are transformed into ectoderm (cerebral cortex, midbrain, hypothalamus, etc.), mesoderm (yolk sac, etc.) and endoderm cells, as well as a process in which hematopoietic stem cells are transformed into red blood cells, white blood cells, platelets, and the like, that is, a process in which progenitor cells express specific differentiation traits.

The existing method of inducing differentiation directly from human pluripotent stem cells to differentiate them into the corresponding cells has a problem in reproducibility depending on the cell line or laboratory (experiment environment), and does not achieve stable expression of maintenance factors. For example, in the case of oligodendrocytes, the development time of oligodendrocytes is the slowest among other nervous system cells even *in vivo,* and an appropriate mass differentiation method has not been developed up to now, so there is a limit to the amount of final cells that may be obtained through one differentiation. As in one embodiment of the present invention, by patterning and preparing ventral neural tube organoids capable of sufficiently containing oligodendrocyte progenitor cells (target cell enriched), that is, obtaining organoids containing the maximum amount of each target cell, and chopping the organoid tissues to culture the corresponding stem cells or progenitor cells, as compared to the two-dimensionally differentiation induced cell population, they may be more similar to the cells isolated from the actual brain, their properties may be well maintained, and it is possible to obtain differentiation into a cell population in which viability is obtained.

As used herein, the term "patterning" refers to the preparation of an organoid such that, when preparing an organoid, the cell population with fate having the property of the origin tissue of the cell to be finally extracted from among the brain detailed tissues is contained as a plurality of cell populations (target cell enriched). In addition, patterning markers include NKX2.2, SOX10, OLIG2, A2B5, PDGFRa, O4, MBP, and the like, depending on the stage of development.

As used herein, the term "chopping" refers to physically (for example, using a needle, etc.) cutting the 3D organoids prepared before subculture into several pieces and dispersing them.

Therefore, according to one embodiment of the present invention, it includes a method, which chops the 3D organoids prepared from human pluripotent stem cells to obtain a large quantity of oligodendrocyte progenitor cells and finally differentiates them, comprising the steps of
1) proliferating and culturing human pluripotent stem cells to prepare 3D organoids;
2) patterning and chopping the prepared 3D organoids; and
3) culturing and proliferating oligodendrocyte progenitor cells in the cells extracted from the chopped organoids, and inducing differentiation in a large quantity to obtain a large quantity of the finally differentiated oligodendrocytes.

As used herein, the term "large quantity" refers to an amount increased by about 100 to 130 times by preparing (25 to 30) 3D organoids when introduced from the start of one culture dish of pluripotent stem cells used for the first time, and chopping the organoids to obtain viable cells, and culturing them for 5 passages. In particular, it includes not only simple quantitative proliferation, but also maintenance of properties.

As used herein, the term "oligodendrocyte progenitor cells" are formed in the ventricular germinal zone of the embryonic neural tube, migrate to another area, differentiate into oligodendrocytes in the arrived nerve area, and then form myelin sheaths for the surrounding axons.

As used herein, the term "oligodendrocyte" refers to an oligodendrocyte made from an oligodendrocyte progenitor cell. Branches extending from oligodendrocytes form myelin sheaths surrounding the axons of neurons around them, and one oligodendrocyte sometimes surrounds 50 different axons. It is closely related to nerve cells and, like other neuroglia, plays a role in supporting neurons.

The present invention also includes a cell therapeutic agent comprising oligodendrocytes obtained by the above method.

"Cell therapeutic agent" is a drug used for treatment, diagnosis, and prevention with cells and tissues prepared through isolation, culture, and special modification from a subject (U.S. FDA regulations). It refers to a drug used for the purpose of treatment, diagnosis, and prevention through a series of actions such as proliferating or sorting living autologous, allogeneic, or xenogeneic cells *ex vivo,* or changing the biological properties of cells in other ways to restore the function of cells or tissues. Cell therapeutic agents are largely classified into somatic cell therapeutic agents and stem cell therapeutic agent according to the degree of cell differentiation.

As used herein, the term "subject" may be a vertebrate to be treated, observed or experimented, preferably a mammal, for example, cattle, pigs, horses, goats, dogs, cats, rats, mice, rabbits, guinea pigs, human, and the like.

As used herein, the term "treatment" refers to any action that inhibits, alleviates, or advantageously alters a clinical situation related to a disease. In addition, treatment may mean increased survival compared to the expected survival rate when not receiving treatment. Treatment simultaneously includes prophylactic means in addition to therapeutic means.

The cell therapeutic agent of the present invention exhibits a therapeutic effect on a nervous system inflammatory disease caused by oligodendrocytopathy.

The nervous system inflammatory disease caused by oligodendrocytopathy may include, for example, any one selected from the group consisting of multiple system atrophy (MSA), multiple sclerosis, cerebral palsy, spinal cord injury, stroke, Lewy body dementia and alpha-synucleinopathy, but is not limited thereto.

The oligodendrocytes obtained by the method of the present invention may be applied as a cell therapeutic agent, and may be formulated by further including a pharmaceutically acceptable carrier. As used herein, the term "pharmaceutically acceptable carrier" refers to a carrier or diluent that does not considerably stimulate the organism and does not inhibit the biological activity and properties of the administered component. In the present invention, the pharmaceutically acceptable carrier that may be included in a cell therapeutic agent may be used without limitation as long as it is known in the art, such as a buffering agent, a preservative, an analgesic agent, a solubilizer, an isotonic agent, a stabilizer, a base, an excipient, a lubricant, and the like. The cell therapeutic agent of the present invention may be prepared in the form of various formulations according to commonly used techniques. The cell therapeutic agent of the present invention may be administered through any route as long as it may induce movement to the diseased site. In some cases, a method of loading the stem cells into a vehicle equipped with a means for directing the lesion may be also considered. Therefore, the cell therapeutic agent of the present invention may be administered through several routes including topical (including buccal, sublingual, dermal and intraocular administration), parenteral (including subcutaneous, intradermal, intramuscular, instillation, intravenous, intraarterial, intraarticular and intracerebrospinal fluid) or transdermal administration, and is preferably administered directly to the site of disease. In one embodiment, the cells may be administered to an individual by suspending the drug in a suitable diluent, and the diluent is used to protect and maintain the cells and to facilitate use when injected into a target tissue. The diluent may include physiological saline, a phosphate buffer solution, a buffer solution such as HBSS, cerebrospinal fluid, and the like. In addition, the pharmaceutical composition may be administered by any device to allow the active substance to migrate to the target cell. A preferred mode of administration and preparation are injections. Injections may be prepared using aqueous solutions such as physiological saline, Ringer's solution, Hank's solution or sterilized aqueous solution, vegetable oils such as olive oil, higher fatty acid ester such as ethyl oleic acid, and non-aqueous solvents such as ethanol, benzyl alcohol, propylene glycol, polyethylene glycol or glycerin, and the like. For mucosal penetration, a non-penetrating agent known in the art suitable for a barrier to pass through may be used, and may further include a pharmaceutical carrier such as ascorbic acid, sodium hydrogen sulfite, BHA, tocopherol, EDTA, and the like as a stabilizer for preventing deterioration, and an emulsifier, a buffering agent for pH adjustment, a preservative for inhibiting the growth of microorganisms such as phenylmercuric nitrate, thimerosal, benzalkonium chloride, phenol, cresol, and benzyl alcohol.

The present invention also provides a drug screening method using the oligodendrocytes obtained by the method.

Important features of the oligodendrocytes obtained by the present invention include the possibility of obtaining the production of a large quantity of cells, the maintenance of their properties even during cryopreservation, the possibility of maintaining the same cell population for a long period of time, and differentiation more similar to those of cells derived from a living body. This property is particularly suitable for simultaneous screening of multiple drugs, which requires a large quantity of cells in the same state and is the key to obtaining the same cells for a long period of time for repeated analysis thereof. It is very suitable for screening cells because a cell population with the same features in which key markers are maintained may be used continuously from the beginning to the end of the screening.

The drug is a drug for treating a nervous system inflammatory disease caused by oligodendrocytopathy, and exhibits a therapeutic effect on the oligodendrocytopathy disease.

The nervous system inflammatory disease caused by oligodendrocytopathy may include, for example, various degenerative nervous system diseases comprising multiple system atrophy (MSA), multiple sclerosis, cerebral palsy, spinal cord injury, stroke, Lewy body dementia and alpha-synucleinopathy, but is not limited thereto.

All technical terms used in the present invention, unless otherwise defined, have the same meaning as commonly understood by one of ordinary skill in the art to which the present invention belongs. In addition although preferred methods and samples are described herein, ones similar or equivalent thereto are also included in the scope of the present invention. The contents of all publications described herein as a reference are incorporated herein by reference.

### Mode for Carrying out the Invention

Hereinafter, the present application will be described in detail through examples. The following examples are only for illustrating the present application, and the scope of the present application is not limited to the following examples.

### [Example]

### Example 1: Differentiation from human induced pluripotent stem cells into mDA (midbrain dopamine) neurons using midbrain organoids

### [Experimental method]

### Culturing of human embryonic stem cells or human induced pluripotent stem cells

hESCs and hiPSCs were cultured based on the hESC research guidelines approved by the institutional review board (IRB) of Hanyang University (Seoul, Republic of Korea). The hESCs and hiPSCs used in this experiment are shown in Table 1 below.

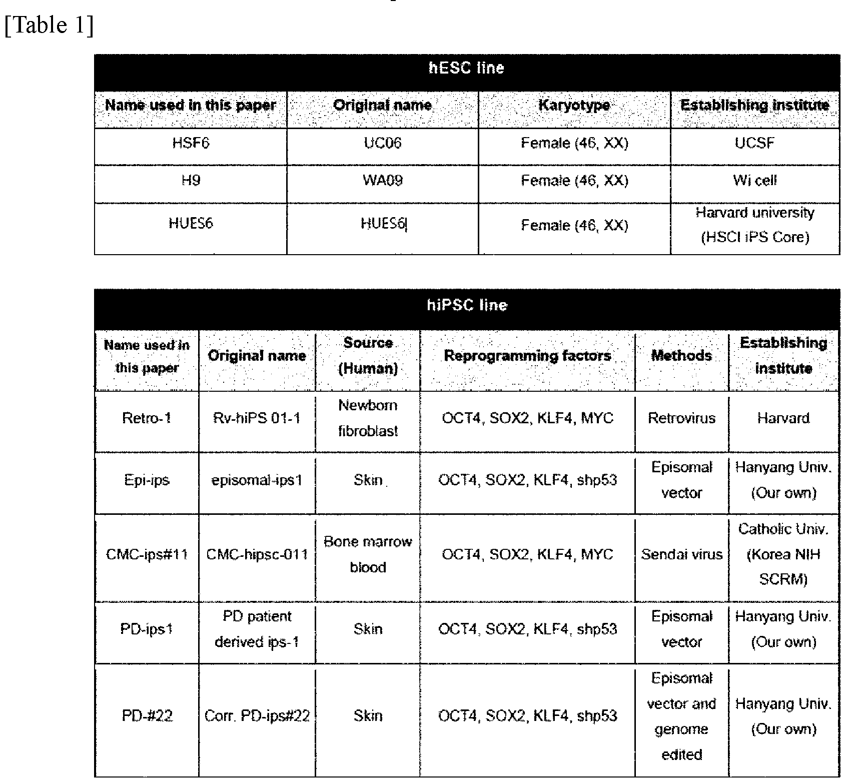

For proliferation and maintenance of undifferentiation hESC/iPSC, the cells were cultured on Matrigel^{™} or on a vitronectin (Human; Gibco Fisher Scientific, Waltham, MA) (Gibco A31804; 0.5 µg/cm²)-coated 6 cm dish (Thermo Fisher Scientific, Waltham, MA) using mTESR-1 medium (Stemcell Technologies Inc., Vancouver, BC, Canada) in a CO₂ incubator set at 37°C without a feeder layer, and medium replacement was performed daily. The undifferentiated stem cells maintained their differentiation ability by replacing the medium daily, and were subcultured using Acutase (Stemcell Technologies Inc.) every 4 to 5 days.

### Preparation of oligodendrocyte progenitor cells using 3D organoid preparation method

Briefly, a system was used in which a ventral 3D organoid was first prepared, cut into small pieces, and proliferated in a large quantity in a culture dish in a state of oligodendrocyte progenitor cells.

The normally cultured human embryonic stem cells (hESC) or human pluripotent stem cells (hiPSC) were detached using Accutase^{™} and counted, and then the cells were suspended into the culture solution on Day 0 of Table 2 below so that there were 10,000 cells in 150 µl. Thereafter, 150 µl (10,000 cells/well) of the Day 0 culture solution in which the cells were released was seeded into each low binding 96-well plate. Thereafter, on the day when the composition of the culture solution was replaced, the culture solution was replaced according to the date, and when the composition of the culture solution was the same, the culture solution was replaced every other day. The culture solution was replaced by 150 µl according to the composition of Tables 2 and 3. On Day 7, the organoids were transferred to a low binding 6 well plate by 8 pieces/well. When transferring, the tip was inserted into a 1000 µl pipette, and then the edge of the tip was cut with sterilized scissors, and the organoids were grabbed with that part and transferred to a low binding 6-well plate. Thereafter, while rotating the low binding 6-well plate in an orbitary shaker (80 to 100 rpm), the culture solution was replaced by 3 ml per well on the day when the composition of the culture solution was changed, or every other day. On Day 36, the organoids were physically cut (chopping) with a 30 G needle, and then the edge of a 60 mm dish coated with poly-l-ornithine and fibronectin was sucked, and the cells were 2D plated in a circle with a diameter of about 4 cm in the center. After the first plating, 4 × 10⁶ cells were cultured in plane in a 60 mm culture dish coated with PLO/FN or poly-l-ornithine and fibronectin using Accutase^{™} once a week for subculture when the cells were about 80 to 90% full. Culture was performed using D36∼ culture solution in Table 2 as a culture medium, and a medium to which mitogen (bFGF 20 ng/ml, PDGF-AA 10 ng/ml, EGF 20 ng/ml) was added was used as a proliferation medium. This process is a step in which a large quantity of oligodendrocyte progenitor cells can be obtained by proliferating and culturing, and it is a step in which the cells can be stored frozen as a stock. Thereafter, when induction was desired through the differentiation process, it was replaced with the differentiation culture solution in Table 2.

**[Table 2]**

| | |
|---|---|
| Day 0 | A media (recipe below) + SB431542 (10 µM, Tocris, Bristol, UK) + Noggin (200 ng/ml, Peprotech, Rocky Hill, NJ) + Y27632 (Sigma, #Y0503, 20 µM) |
| Day 1∼2 | A media (recipe below) + SB431542 (10 µM, Tocris, Bristol, UK) + Noggin (200 ng/ml, Peprotech, Rocky Hill, NJ) + Sonic hedgehog (100 ng/ml, SHH, Peprotech) + purmorphamine (1 µM, Calbiochem, MilliporeSigma, Burlington, MA) |
| Day 3∼4 | A media (recipe below) + SB431542 (10 µM, Tocris, Bristol, UK) + Noggin (200 ng/ml, Peprotech, Rocky Hill, NJ) + Sonic hedgehog (100 ng/ml, SHH, Peprotech) + purmorphamine (1 µM, Calbiochem, MilliporeSigma, Burlington, MA) + Retinoic acid (0.1 µM, Sigma-Aldrich, #R2625) |
| Day 5∼11 | A media (recipe below) + Noggin (200 ng/ml, Peprotech, Rocky Hill, NJ) + Sonic hedgehog (100 ng/ml, SHH, Peprotech) + purmorphamine (1 µM, Calbiochem, MilliporeSigma, Burlington, MA) + Retinoic acid (0.1 µM, Sigma-Aldrich, #R2625) |
| Day 12∼16 | A media (recipe below)+ Sonic hedgehog (100 ng/ml, SHH, Peprotech) + purmorphamine (1 µM, Calbiochem, MilliporeSigma, Burlington, MA) + Retinoic acid (0.1 µM, Sigma-Aldrich, #R2625) |
| Day 17 | A media (recipe below)+ Sonic hedgehog (100 ng/ml, SHH, Peprotech) + purmorphamine (1 µM, Calbiochem, MilliporeSigma, Burlington, MA) + bFGF (20 ng/ml, R&D Systems) + EGF (10 ng/ml, R&D Systems) |
| Day 18∼35 | B media (recipe below) + bFGF (20 ng/ml, R&D Systems) + EGF (10 ng/ml, R&D Systems) |
| Day 36∼ | B media (recipe below) + bFGF (20 ng/ml, R&D Systems) + PDGF-AA (10 ng/ml, R&D Systems, #221-AA) + EGF (10 ng/ml, R&D Systems) |
| Differentiation day 0∼14 | N2 media + T3 (60 ng/ml, Sigma-Aldrich, #T0281) + db-cAMP (1 µM, Sigma-Aldrich, #D0627) + Biotin (100 ng/ml, Sigma-Aldrich, #B4639) + PDGF-AA (10 ng/ml, R&D Systems, #221-AA) + IGF-1 (10 µg/ml, peprotech, #110-11) + NT3 (10 µg/ml, peprotech, #450-03) + Ascorbic Acid (200 µM, Sigma, St. Louis, MO) + insulin (Gibco, #12585014, 0.31 µl/ml) |
| Differentiation day 15∼ | N2 media + T3 (60 ng/ml, Sigma-Aldrich, #T0281) + cAMP (1 µM, Sigma-Aldrich, #D0627) + Biotin (100 ng/ml, Sigma-Aldrich, #B4639) + IGF-1 (10 µg/ml, peprotech, #110-11) + NT3 (10 µg/ml, peprotech, #450-03) + Ascorbic Acid (200 µM, Sigma, St. Louis, MO) + insulin (Gibco, #12585014, 0.31 µl/ml) |

**[Table 3]**

| **<A media>** | |
|---|---|
| 2X N2 meida(50%) | |
| | -2X N2 recipe as follows(1L) |
| | D.W. 1L |
| | DMEM-F12 12g |
| | Sodium Bicarbonate 1.69g |
| | D-glucose 1.55g |
| | I,-Glutamine 0,073g |
| | Apo-transferrin 100mg |
| | Putrescine 200µl |
| | Selenite 120µl |
| | Progesterone 400µl |
| Neurobasal Medium (50%) (Neurobasal, Gibco, #211030-19) | |
| B27(Gibco, #17504044, 1:50) | |
| insulin (Gibco. #12585014. 0.31µl/ml) | |

| **<B media>** | |
|---|---|
| N2 meida(100%) | |
| | -N2 recipe as follows(1L) |
| | D.W. 1L |
| | DMEM-F12 12g |
| | Sodium Bicarbonate 1.69g |
| | D-glucose 1.55g |
| | L-Glutamine 0.073g |
| | Apo-transferrin 50mg |
| | Putrescine 100µl |
| | Selenite 60µl |
| | Progesterone 200µl |
| B27(Gibco. #17504044. 1:50) | |
| insulin (Gibco, #12585014. 0.31µl/ml) | |

### Immunocytochemistry analysis

Immunocytochemistry analysis is an analysis method that visualizes the target protein by attaching a primary antibody to the target protein and then attaching a secondary antibody to which fluorescent dye is attached to that antibody. The number of proteins that can be visualized is determined by the type of fluorescence of the secondary antibody. In this study, two or three types of fluorescent dye were used.

Sample was fixed with a fixative solution (4% paraformaldehyde/PBS) for 20 minutes, and then rinsed three times with PBS for 5 minutes each time. Blocking was performed for 40 minutes using a blocking buffer (1% BSA/PBS, 0.1% Triton X100), and then the primary antibody was dissolved in the same buffer and attached to the sample for 24 hours. The sample was rinsed three times with 0.1% BSA/PBS, and then the secondary antibody was dissolved in the same buffer and attached for 1 hour. Thereafter, the sample was rinsed three times with 0.1% BSA/PBS, and then rinsed once with D.W., and mounted on a cover slide with a mounting solution (Vectashield, Vector Lab).

The organoid section immunocytochemistry analysis was performed as follows. The organoid was fixed in a fixative solution for more than 30 minutes, and then rinsed three times with PBS for 10 minutes each time, and then cultured in a 30% sucrose solution for 24 hours, and dehydrated. The dehydrated sample was frozen in an OCT (optimal cutting temperature) compound and then cut to a thickness of 10 to 12 µm with a microtome, and the above blocking process was performed.

### Cryosection of organoids

The 1000 µl tip that was cut slightly in front thereof was inserted into a pipette, and the organoids that were contained in the culture dish were grabbed with that part and transferred to a 15 ml tube containing 1 ml of 4% PFA. The organoids were fixed at room temperature for 15 minutes, and then 4% PFA was discarded and washed three times repeatedly with 3 ml of PBS for 15 minutes each time. Thereafter, all the washed solutions were discarded, 3 ml of sucrose solution was added, and the organoids were submerged while refrigerated for about 3 days. After 3 days, it was confirmed that the organoids were submerged, and a cylindrical frame with a hole in the top was made with foil. Thereafter, the OTC compound was poured into the frame made with foil, and the organoids submerged in the sucrose solution were transferred into the frame made with foil using the 1000 µl tip that was cut slightly in front thereof and a pipette. After keeping them in the deep freezer for more than 3 hours, the foil was peeled off while keeping the temperature cold. Thereafter, the OCT compound was sprinkled on the chuck enough to cover the surface, and the frozen organoids were attached to the chuck in a cylindrical shape with the foil peeled off. When the two organoids were attached, the OCT compound was sprinkled all over once again, and the organoids were frozen in the deep freezer for more than 3 hours again, and then cryostat was performed. The thickness of the cryosta was 12 to 18 mm.

### qPCR

RNA of the sample to be analyzed was extracted using Trizol, and after RNA extraction was completed, cDNA was synthesized, and RT-PCR was performed. This is an experimental method that can compare the relative amount of RNA present between samples, and in this study, it was used to confirm the expression of a marker protein in a specific sample through comparison between several samples.

The RNA extraction method is as follows.

The cells were lysed in 1 ml of Trizol for 5 minutes, and 200 µl of chloroform was added, mixed with shaking, and then let stand for 2-3 minutes. Centrifugation was performed at 12000 Xg at 4°C for 15 minutes. After centrifugation, the transparent part of the supernatant was collected (400 to 500 µl) and transferred to a new tube, and 500 µl of isopropanol was added thereto and then mixed. After incubation for 10 minutes, centrifugation was performed at 12000 Xg at 4°C for 10 minutes.

The supernatant was removed except for a small amount of RNA mass that remained at the bottom of the tube, and then 1 ml of 75% ethanol solution was added and mixed with the mass. Centrifugation was performed at 7500 Xg at 4°C for 5 minutes. The supernatant was removed, and then only the RNA mass was left, and then the lid was opened and air-dried. The dried RNA was dissolved in RNase-free water, and then the concentration thereof was measured.

The cDNA synthesis method is as follows.

2 µg of RNA was used for cDNA synthesis. First-strand cDNA synthesis was performed using a random primer (Invitrogen), and reaction was performed by a PCR machine at 75°C for 15 minutes. Thereafter, incubation was performed on ice for 2 minutes, and then first-strand buffer (Invitrogen), DTT (Invitrogen), and RNasin (Promega) were added, and reaction was performed at 25°C for 15 minutes, at 42°C for 50 minutes, and at 70°C for 15 minutes.

The prepared cDNA was a total of 20 µl, which was diluted 10-fold and used for RT-PCR. SYBR green master mix (Bio-rad) was used for the reaction, and 2 µl of cDNA was reacted each time.

### Mass proliferation of 3D organoid derived oligodendrocyte progenitor cells

For the mass proliferation of oligodendrocyte progenitor cells, the organoids that were physically cut (chopping) with a 30 G needle on Day 36 were 2D plated on a 60 mm dish coated with poly-l-ornithine and fibronectin in a circle with a diameter of about 4 cm in the center. After the first plating, 4 × 10⁶ cells were cultured in plane in a 60 mm culture dish coated with PLO/FN or poly-l-ornithine and fibronectin using Accutase^{™} once a week for subculture when the cells were full. Culture was performed using D36∼ culture solution in Table 2 as a culture medium, and a medium to which mitogen (bFGF 20 ng/ml, PDGF-AA 10 ng/ml, EGF 20 ng/ml) was added was used as a proliferation medium. This process is a step in which a large quantity of oligodendrocyte progenitor cells can be obtained by proliferating and culturing.

### Frozen storage of 3D organoid derived oligodendrocyte progenitor cells

When cells were obtained in a 60 mm culture dish through mass proliferation of oligodendrocyte progenitor cells, these cells could be detached and stored frozen. For the frozen storage method, the cells were subcultured and cultured in D36∼ culture solution to which mitogen was added in Table 2 for at least one week. Thereafter, the cells were detached using Accutase^{™}, and the number of cells was determined through counting. 6 × 10⁶ cells per vial were resuspended in 1 ml of a frozen storage solution with a composition composed of 10% DMSO and 90% D36∼ culture solution, and then placed in a cryotube, and stored frozen.

### Transduction of lentivirus (pEF1α-α-syn-GFP)

A lentivirus expressing a-syn-GFP was added to D36∼ culture solution at 100X, and the cells were treated overnight. The next day, the culture solution was replaced with the original D36∼ culture solution.

### Comparison of difference between synuclein monomer and a-synclein aggregate of oligodendrocyte progenitor cells

The oligodendrocyte progenitor cells were transduced with lentivirus (pEF1α-α-syn-GFP) and then were cultured in D36∼ culture solution for 5 days. Thereafter, differentiation was carried out for 7 days with the culture solution of differentiation day 0-14. On day 7, the cells were washed with PBS, and then the cells were treated with 150 µl of Ripa buffer. After treating the cells, they were incubated on ice for about 5 minutes. Thereafter, sonication was carried out for about 5 seconds, the proteins were quantified, and then the amount of the proteins was adjusted to 10 µg, and the amount of monomer was adjusted to 300 ng. They were treated with Proteinase K at 0.12 U, 0.6 U, and 3 U, respectively. Thereafter, shaking was carried out at 37°C for 1 hour. After incubation, 5X sample buffer was added, incubation was carried out in a heat block at 95°C for 10 minutes, and then samples were loaded on 15 mm, 15% SDS gel, and western blot was performed.

### Analysis of intercellular α-syn uptake

After differentiation of each cell type for 7 days, the culture solution was treated with PFF at a concentration of 1 µg/ml overnight. Fluorescent dye was attached by PFF using alexa fluor 488 microscale protein labeling kit. The intensity was measured using a fluorescence microscope at intervals of one day or two days from D0, and the number of cells that uptook PFF was counted.

### Detection of pathological α-syn aggregates

Each cell was differentiated in a 12 well plate for 8 days and then treated with PFF at a concentration of 2 µg/ml overnight. On D0 (before the cells were treated with PFF), D1, D3, and D11 after PFF treatment, 25 µl of the culture solution was collected and kept in a deep freezer. Upon experiment, it was thawed, treated with 5X sample buffer, and loaded on the SDS gel. The cell samples were collected using 50 µl of 1% triton X-100, 1% protease inhibitor cocktail in PBS solution on the same day. The cell samples were collected, and then incubated on ice for 15 minutes, and then they could be stored in a deep freezer, and thawed again during the experiment, and centrifuged at 4°C at 16,000xg for 10 minutes. The supernatant was transferred to a separate 1.5 ml tube in soluble form and quantified by BCA assay. The remaining pellet was in insoluble form and sonicated by adding 25 µl of 1X sample buffer. 10 µg of the soluble form protein based on the quantified protein concentration of the soluble form was mixed with 5X sample buffer and loaded on the SDS gel, and 10 µg of the insoluble form protein based on the amount of the soluble form protein was loaded on the gel, and western blot was performed.

### [Experimental result]

### Confirmation of preparation of ventral patterning organoids from human pluripotent stem cells

Confirmation of preparation of ventral patterning organoids (ventral neural tube), which were specifically generated in such a way that they can contain a large number of oligodendrocyte progenitor cells (OPCs), was carried out by measuring the size through an optical microscope. Normal culture of the organoids can be confirmed by size. The organoids were cultured with an average size of 1.6 mm to 1.8 mm, and it was determined that the organoids outside this range were organoids that were not normally cultured.

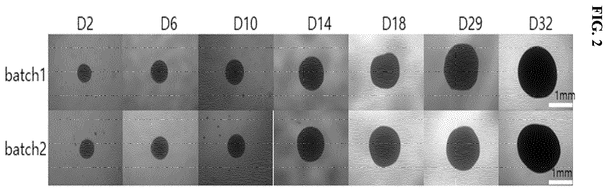

As shown in FIG. 2, it can be confirmed that the normally grown ventral patterning organoids with a size of 1.6 mm to 1.8 mm was prepared.

### Confirmation of mRNA expression in oligodendrocyte progenitor cells by qPCR of patterned organoids

The mRNA expression level of oligodendrocyte progenitor cells in the organoids was confirmed by qPCR. It was confirmed that the organoids were prepared so that the cell population with fate having the properties of the origin tissue of the cell was contained as a plurality of cell populations (target cell enriched), and it was confirmed that the expression of NKX2.2, SOX10, OLIG2, A2B5, PDGFRa, O4, MBP, and the like was increased according to the patterning marker generation stage.

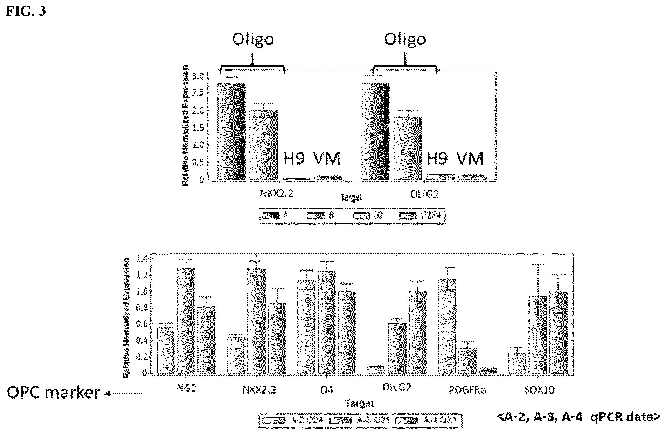

As shown in FIG. 3, NKX2.2, a marker of oligodendrocyte progenitor cells, was expressed 150 times higher and OLIG2 was expressed 20 times higher than that of H9, human embryonic stem cells.

### Photograph of staining of ventral patterning organoids, which were specifically generated in such a way that they can contain a large number of oligodendrocyte progenitor cells (OPCs)

The organoids that were pattern cultured for 21 days were cryosectioned, and then a representative oligodendrocyte progenitor cell marker (oligodendrocyte progenitor marker) was confirmed through immunostaining. Briefly, the OCT compound was sprinkled on the chuck, which was cryogenically frozen in a deep freezer for 3 days, enough to cover the surface, and the frozen organoids were attached to the chuck. When the two organoids were attached, the OCT compound was sprinkled all over once again, and the organoids were frozen in the deep freezer for more than 3 hours again, and then cryostat was performed. The thickness of the cryosta was 12 to 18 mm. The marker was analyzed by fluorescence immunoassay.

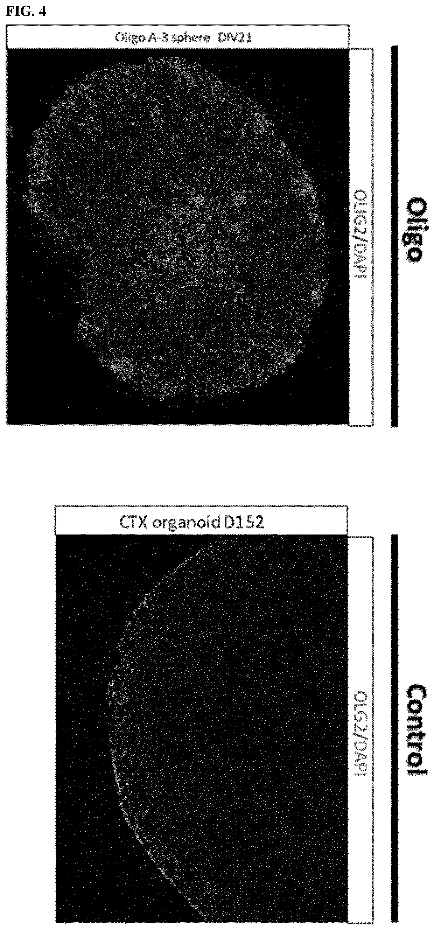

As shown in the left of FIG. 4, it was confirmed that OLIG2, an oligodendrocyte progenitor cell marker, was expressed much more in the organoid of the present invention than in the cortex organoid (right), a negative control group.

### Obtaining a large quantity of oligodendrocytes

After converting the organoids into a 2D culture environment through a physical chopping process, the number of the proliferated cells and markers of oligodendrocyte progenitor cells and oligodendrocytes were confirmed.

On Day 36, the organoids were physically cut (chopping) with a 30 G needle, and then the edge of a 60 mm dish coated with poly-l-ornithine and fibronectin was sucked, and the cells were 2D plated in a circle with a diameter of about 4 cm in the center. After the first plating, 4 × 10⁶ cells were cultured in plane in a 60 mm culture dish coated with PLO/FN or poly-l-ornithine and fibronectin using Accutase^{™} once a week for subculture when the cells were about 80 to 90% full. Culture was performed using D36∼ culture solution in Table 2 as a culture medium, and a medium to which mitogen (bFGF 20 ng/ml, PDGF-AA 10 ng/ml, and EGF 20 ng/ml) was added was used as a proliferation medium. The cells were counted at each step during passage.

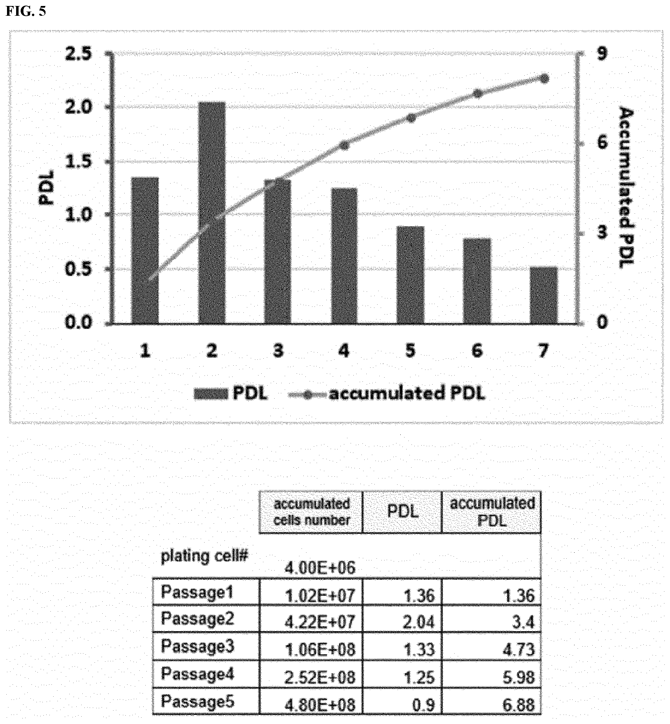

The organoids were physically chopped with a 30 G needle and converted to a two-dimensional 2D culture environment in a 6 mm PLO/FN-coated dish, and then the numbers of the proliferated PDL (population doubling level) and accumulated PDL were measured through subculture. Compared to other protocols that cannot proliferate at the oligodendrocyte progenitor cell stage, it was confirmed that this protocol can proliferate about 120-fold cells based on 5 passages since the first plating [FIG. 5].

(When 25 to 30 organoids are chopped, about 4×10^6 cells can be obtained based on only living cells, not dead cells. These cells are spread in a 60 mm dish, and when cultured for 5 passages starting from here, the number of cells increases by 120 times, reaching the number of about 4.8×10^8 cells. When making a frozen vial of cells, 6×10^6 cells are put in 1 vial, so 80 vials of cell stock can be made with cells obtained up to 5 passages.)

### Confirmation of differentiation into oligodendrocytes after frozen storage of oligodendrocyte progenitor cells

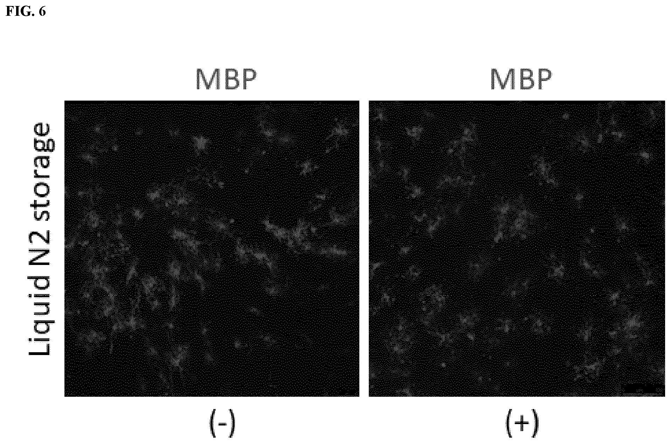

As shown in FIG. 6, it was confirmed that when oligodendrocyte progenitor cells were induced to differentiate after thawing compared to before cryopreservation (left), their differentiation ability was maintained and differentiated into oligodendrocytes expressing the marker MBP. The cells were 2D plated, and once a week for subculture when the cells were about 80 to 90% full, culture was performed using D36∼ culture solution in Table 2 as a culture medium, and a medium to which mitogen (bFGF 20 ng/ml, PDGF-AA 10 ng/ml, and EGF 20 ng/ml) was added was used as a proliferation medium. This process is a step in which a large quantity of oligodendrocyte progenitor cells can be obtained by proliferating and culturing, and it is a step in which the cells can be stored frozen as a stock. In the proliferation culture step, oligodendrocyte progenitor cells can be frozen and stored as a stock, so the cells can be used immediately from this step if necessary. This protocol can save at least about 36 days, the time it takes to generate oligodendrocyte progenitor cells from human embryonic stem cells.

### Confirmation of the possibility of proliferation into oligodendrocyte progenitor cells maintaining the ability to differentiate into oligodendrocytes by the marker OLIG2

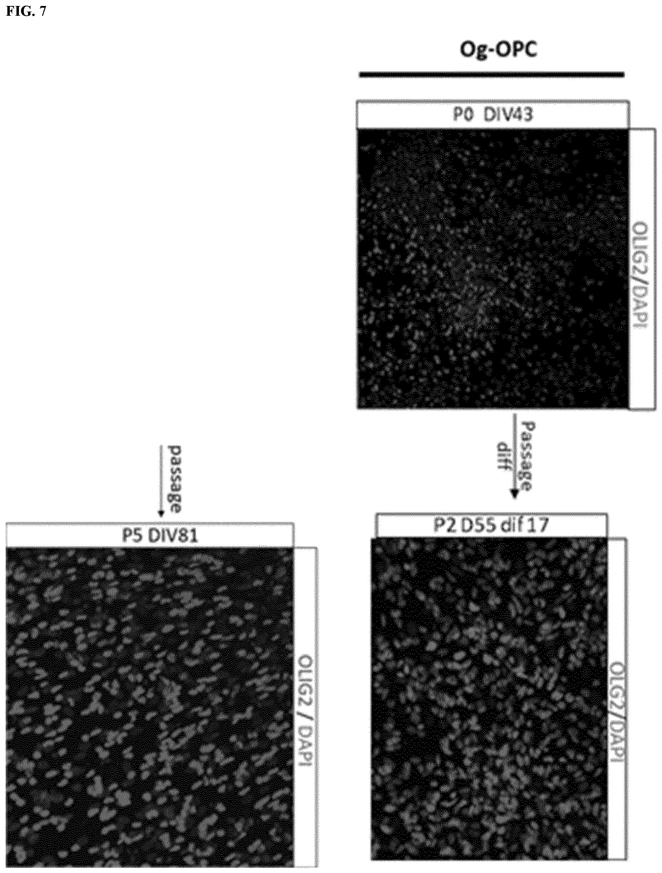

OLIG2, a marker that continues to be expressed from oligodendrocyte progenitor cells to fully differentiated mature oligodendrocytes, continued to be expressed even after the organoids were converted to a 2D culture environment. It was confirmed by immunofluorescence analysis that the expression of OLIG2 was maintained constant even after 5 to 6 passages of culture and differentiation process [FIG. 7].

### Confirmation of expression of oligodendrocyte progenitor cell maintenance marker

It was confirmed by immunofluorescence analysis that A2B5, an initial marker of oligodendrocyte progenitor cells, was mainly expressed in the process of culturing and proliferating oligodendrocyte progenitor cells from Day 36. The cells were 2D plated, and culture was performed using D36∼ culture solution in Table 2 as a culture medium once a week for subculture when the cells were about 80 to 90% full, and a medium to which mitogen was added was used as a proliferation medium. In this process, a large quantity of oligodendrocyte progenitor cells were proliferated and cultured.

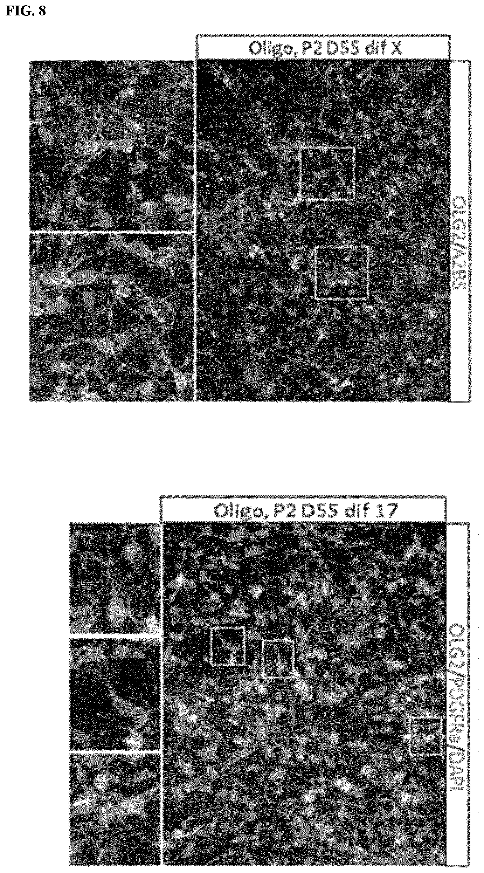

In addition, the marker PDGFRa, a marker of oligodendrocyte progenitor cells, was confirmed in the process of Day 36∼ culture and proliferation or at the initial stage of the differentiation process by replacing the differentiation 0-14 medium [FIG. 8].

### Confirmation of oligodendrocyte differentiation

It was confirmed by the neuron bundle marker NF and the mature oligodendrocyte marker MBP that after differentiation of oligodendrocyte progenitor cells, they normally differentiate into oligodendrocytes, thereby performing the function of myelination of neurons. Culture was performed using D36∼ proliferation culture solution shown in Table 2 above as a culture medium, and the expression in mature oligodendrocytes induced by the subsequent differentiation process was confirmed. For differentiation, it was replaced with the differentiation culture solution in Table 2.

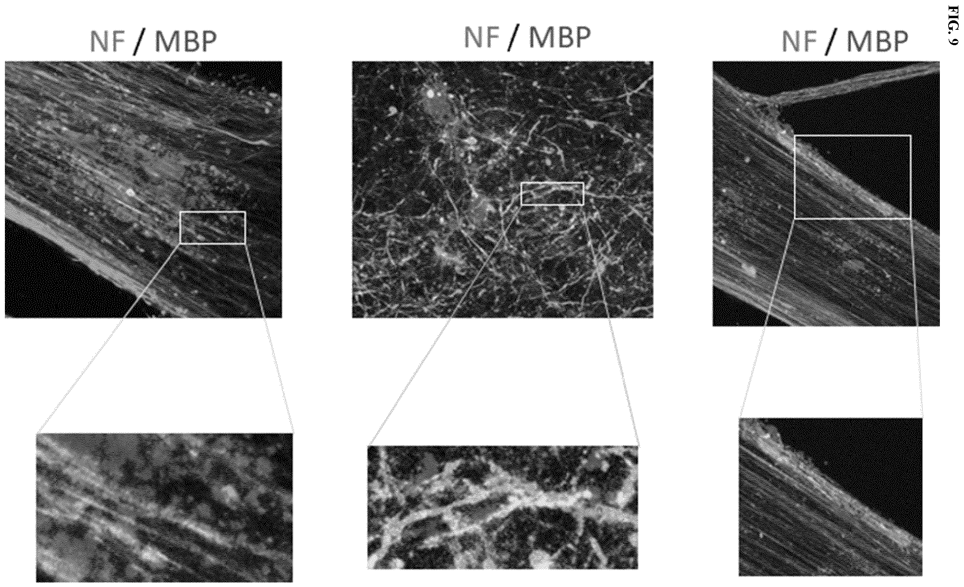

In FIG. 9, it was confirmed that the differentiated mature oligodendrocytes surround the dendrites of neurons. This confirms the fact that the cells were differentiated into differentiated mature oligodendrocytes with a full function of myelination.

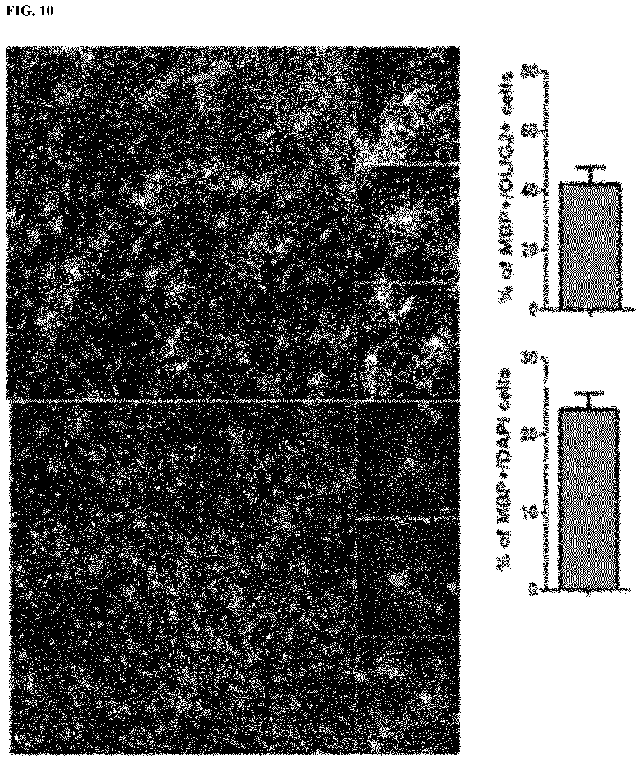

In addition, the expression of O4, an important marker of immature oligodendrocytes, was confirmed after culturing for about 14 days with differentiation day 0∼14 culture solution at a desired time in the proliferation stage of oligodendrocyte progenitor cells [FIG. 10]. Thereafter, after further differentiation for about 2 weeks with differentiation day 15∼ culture, immature oligodendrocytes were completely differentiated into mature oligodendrocytes, and MBP, an important marker of mature oligodendrocytes, was confirmed by fluorescence staining. At this time, it was confirmed that the average ratio of mature oligodendrocytes, the final differentiated cells, was 23% or more compared to the total cells, and 42% or more compared to the OLIG2+ cells. This ratio was confirmed to be a very high ratio compared to the prior art (Non-Patent Documents 1-4).

### Confirmation of disease modeling possibility using generated oligodendrocytes

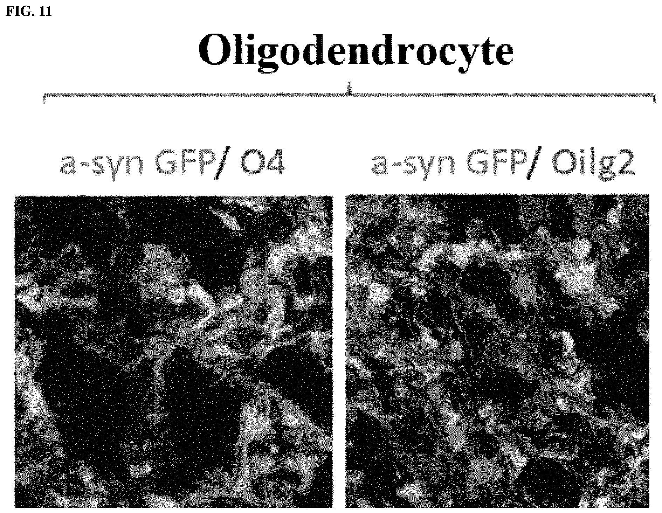

In FIG. 11, it was confirmed by the GFP marker that a lentivirus (pEF1α-α-syn-GFP) overexpressing alpha-synclein (synclein), which can cause synucleinopathy, was well introduced into oligodendrocyte progenitor cells. The cells were 2D plated and subcultured once a week when the cells were about 80% to 90% full. A medium to which mitogen was added was used as a proliferation medium, and virus was introduced into a large quantity of proliferated oligodendrocyte progenitor cells as in the experimental method, and synuclein expression was analyzed by fluorescence analysis.

The oligodendrocyte progenitor cells were transduced with a lentivirus (pEF1α-α-syn-GFP) overexpressing a-synclein, and then each difference was compared.

It was confirmed that a-syn was well overexpressed in the finally differentiated oligodendrocytes. Therefore, it was confirmed that it is a cell that can be used for studying a disease (MSA) caused by a-synuclein aggregation in oligodendrocytes, and the possibility for studying this disease was also confirmed.

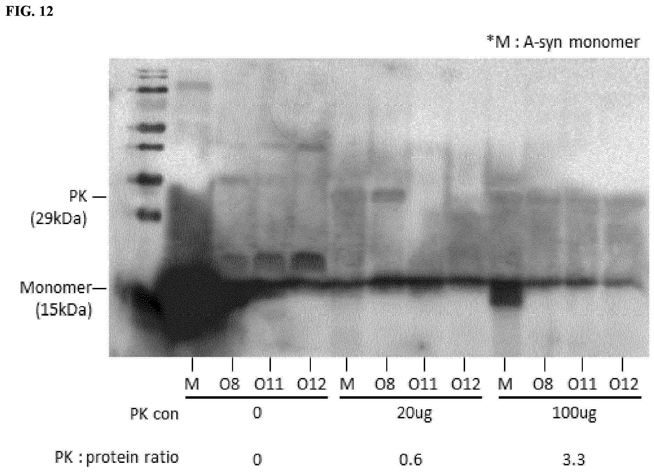

The oligodendrocytes were transduced with a lentivirus (pEF1α-α-syn-GFP), and then the difference in a-synclein aggregate was confirmed by comparison with synuclein monomer (FIG. 12).

Using this, we confirmed the possibility of modeling a disease such as MSA in which synucleinopathy is specifically caused in oligodendrocytes in the future. For the synuclein aggregation model, each cell was differentiated in a 12 well plate for 8 days and then treated with PFF at a concentration of 2 µg/ml overnight. For analysis, the protein of the cells was collected, and then treated with 5X sample buffer, and loaded on the SDS gel.

The oligodendrocytes were transduced with a lentivirus (pEF1α-α-syn) overexpressing a-synuclein to overexpress a-synuclein and then treated with a-synuclein monomer (M) and proteinase K, respectively. The difference in PK digestion was observed.

In the group with a PK concentration of 0, the monomer and aggregates of oligodendrocytes (O8, O11, and 012) were not cleaved by the enzyme. However, it was observed that as the concentration of PK increased, the aggregate was cleaved to become a monomer. In the group with the highest concentration, it was observed that the monomer was cleaved to a smaller size, but the oligodendrocytes were not cleaved to a smaller size.

An experiment was conducted to observe a pattern of uptake of a-synuclein pre-formed fibrils (PFF) by day for each cell type in OPC (O, oligodendrocyte) compared to N (neuron) and A (astrocyte), respectively.

The oligodendrocyte progenitor cells were differentiated into oligodendrocytes with differentiation day 0∼14 culture solution for about 2 weeks, and the oligodendrocytes were sorted by MACS to increase the purity and then differentiated for about 7 days with other types of cells (N-neuron, A-astrocyte, and O-oligodendrocyte), respectively. Thereafter, Alexa 488-attached PFF was mixed with the culture solution at a concentration of 1 µg/well, and the cells were treated therewith for one day. The next day, the culture solution was replaced with a normal culture solution, and then the cells and the fluorescence expressed in the cells by date was compared by taking pictures.

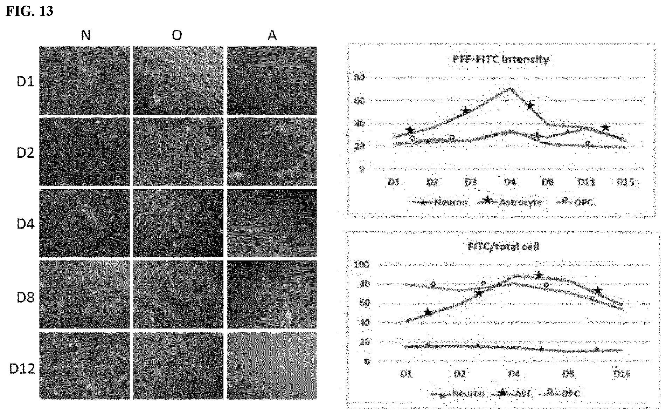

As shown in FIG. 13, astrocytes not only uptake the most PFF, but also phagocytose PFF over time. However, it was confirmed that oligodendrocytes uptake PFF but do not have the ability to phagocytose. When oligodendrocytes uptake PFF, a-synuclein is aggregated in oligodendrocytes only by PFF treatment. Accordingly, this makes it possible to conduct experiments for the study of pathologies in which a-synuclein is aggregated in oligodendrocytes. In order to confirm the phagocytosis ability of the medium, on D0 (before the cells were treated with PFF), D1, D3, and D11 after PFF treatment, 25 µl of the culture solution was collected and kept in a deep freezer. Upon experiment, it was thawed, treated with 5X sample buffer, and loaded on the SDS gel.

In order to confirm the PFF uptake of oligodendrocytes and the degree of a-syn aggregation in oligodendrocytes, the cells were differentiated for each cell type for 8 days and then, the culture solution was treated with PFF at a concentration of 2 µg/ml. Thereafter, in order to confirm a-syn aggregation by PFF, cell function was maintained, western blot samples were collected by date, and the culture solution was also collected by date, and differences were confirmed by western blot.

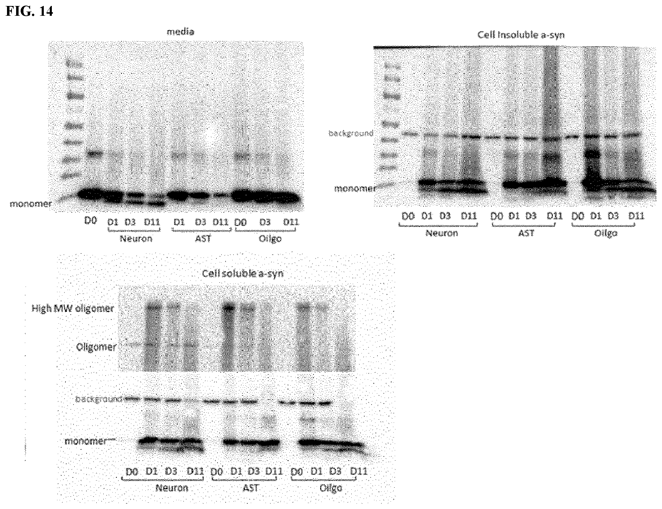

As a result, it was confirmed that oligodendrocytes did not uptake much PFF compared to other cell types in the media data (FIG. 14). However, unlike the amount of PFF uptake, it was confirmed that the amount of a-synuclein in the cell was similar to that of other cells. Therefore, this indicates that phagocytosis is not active, but the oligomerization of a-syn in the cell is good. Therefore, it can be seen that the seed activity of oligodendrocytes is better than that of neurons or astrocytes.

Therefore, it was confirmed that even a small amount of PFF can easily induce a-syncleinopathy in oligodendrocytes. In addition, it was confirmed that this makes it possible to study a-syncleinopathy in oligodendrocytes.

### [Conclusion]

Unlike the prior art (Non-Patent Documents 1-4), the method of the present invention enables proliferation because it includes a proliferation step of oligodendrocyte progenitor cells, and in particular, the proliferative capacity is very strong at the beginning, so a large quantity of cells may be obtained. In addition, at this step, subculture can be performed and the oligodendrocyte progenitor cells can be stored as a stock. Therefore, when necessary, the stored oligodendrocyte progenitor cells can be used immediately, thereby saving time.

In addition, PDGFRa, which is expressed in oligodendrocyte progenitor cells, is expressed up to 15 weeks faster than the prior art. In addition, O4, a marker of immature oligodendrocytes, is expressed rapidly at about 4 to 13 weeks. Since oligodendrocytes are cells that usually take a very long time to differentiate, the culture solution can be saved as much as the shortened time.

## Claims

1. A method for differentiation of oligodendrocytes obtained in a large quantity, comprising:
culturing human pluripotent stem cells to prepare ventral organoids, patterning and chopping the prepared organoids to obtain a large quantity of oligodendrocyte progenitor cells; and
culturing them to differentiate into oligodendrocytes.

2. A cell therapeutic agent comprising oligodendrocytes differentiated by the method according to claim 1.

3. The cell therapeutic agent according to claim 2, wherein the cell therapeutic agent treats a nervous system inflammatory disease caused by oligodendrocytopathy.

4. The cell therapeutic agent according to claim 3, wherein the nervous system inflammatory disease caused by oligodendrocytopathy is any one selected from the group consisting of multiple system atrophy (MSA), multiple sclerosis, cerebral palsy, spinal cord injury, stroke, Lewy body dementia and alpha-synucleinopathy.

5. A drug screening method using oligodendrocytes obtained by the method according to claim 1.

6. The drug screening method according to claim 5, wherein the drug treats a nervous system inflammatory disease caused by oligodendrocytopathy.

7. The drug screening method according to claim 6, wherein the nervous system inflammatory disease caused by oligodendrocytopathy is one selected from the group consisting of multiple system atrophy (MSA), multiple sclerosis, cerebral palsy, spinal cord injury, stroke, Lewy body dementia and alpha-synucleinopathy.
